(19) Europäisches Patentamt
European Patent Office
Office européen des brevets

(11) Publication number: **0 053 408**
**B1**

(12) **EUROPEAN PATENT SPECIFICATION**

(45) Date of publication of patent specification: **17.10.84**

(51) Int. Cl.³: **C 07 C 67/313,**
C 07 C 102/00, C 07 C 51/373,
C 07 D 333/24, C 07 D 213/55

(21) Application number: **81201199.7**

(22) Date of filing: **29.10.81**

(54) **Process for the preparation of substituted glyoxylic acid derivatives.**

(30) Priority: **13.11.80 NL 8006192**

(43) Date of publication of application:
**09.06.82 Bulletin 82/23**

(45) Publication of the grant of the patent:
**17.10.84 Bulletin 84/42**

(84) Designated Contracting States:
**AT BE CH DE FR GB IT LI NL SE**

(56) References cited:
**US-A-1 627 091**
**US-A-2 429 877**

**CHEMICAL ABSTRACTS, vol. 71, no. 26, 19-12-1969, page 405, abstract 129282p Columbus, Ohio, US K. JIJEE et al.: "Oxidation of some carboxylic acids by aqueous solutions of cobalt (III) sulfate"**

(73) Proprietor: **STAMICARBON B.V.**
**Postbus 10**
**NL-6160 MC Geleen (NL)**

(72) Inventor: **Corvers, Antonius**
**Kelmonderstraat 33**
**NL-6191 RD Beek (NL)**
Inventor: **van den Broek, Cornelis Wilhelmus**
**Marie Koenenstraat 8**
**NL-6372 KX Schaesberg (NL)**
Inventor: **Suverkropp, Geertrudes Herman**
**Debijestraat 7**
**NL-6164 BE Geleen (NL)**

(74) Representative: **Hoogstraten, Willem Cornelis Roeland et al**
**OCTROOIBUREAU DSM Postbus 9**
**NL-6160 MA Geleen (NL)**

**EP 0 053 408 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of substituted glyoxylic acid derivatives by oxidation of the corresponding hydroxy derivative.

An example of such a reaction known in the art is the oxidation of a mandelic acid ester to the corresponding ester of phenylglyoxylic acid, which oxidation can be carried out with selenium dioxide or lead tetra-acetate as oxidant (see page 127 of RODD's Chemistry of Carbon Compounds, 2nd edition, volume III part E, 1974, Elsevier Scientific Publishing Company). Another oxidant known in the art for said oxidation is chromium trioxide (see European patent application 0.006.539).

A major disadvantage of the application of these oxidants is that a large quantity of metal compound is obtained as waste.

Now, the invention provides a process in which no metal compound is obtained as waste.

The process according to the invention for the preparation of substituted glyoxylic acid derivatives by oxidation of the corresponding hydroxy derivative is characterized in that an amide or ester of a hydroxy acid with the general formula of

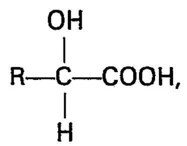

where R represents a possibly substituted aryl or heteroaryl group, is oxidized at a temperature of 70—150°C in a solvent, containing cobalt as catalyst and a bromide as promotor and using a saturated aliphatic monocarboxylic acid with 2—8 C atoms as the solvent, with a molecular oxygen-containing gas, and the corresponding amide or the corresponding ester of a glyoxylic acid with the general formula of

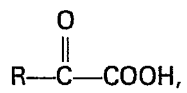

where R has the above-mentioned meaning, is recovered from the reaction mixture obtained.

It has been found that the oxidation according to the invention can be carried out more quickly than the oxidation in which the above-mentioned known oxidants are applied, while, moreover, a higher degree of selectivity can be reached. Furthermore, the product obtained is often pure enough and as such suitable for further conversions, for instance to herbicide.

It is noted that in C.A. Vol. 71, no. 26, page 405, abstract 129282p an article of K. Jijee et al. is mentioned which relates to a kinetic study at 5—25°C of the oxidation of some carboxylic acids, e.g. mandelic acid, by aqueous solutions of cobalt (III) sulfate. Said study does not describe a practical method for the preparation of the glyoxylic acid derivatives according to the invention. Also the method described in US—A—1 627 091, which is related to the preparation of alpha keto aliphatic amides by oxidation of the corresponding hydroxy compound in the gas phase with an oxidation catalyst e.g. cobalt, has no practical significance for the preparation of the glyoxylic derivatives according to the invention because of the low conversion and low yield.

The favourable result in the process according to the invention is very surprising. The fact is that, if the nitrile of the hydroxy acid or the hydroxy acid as such is oxidized in the same manner, a reaction mixture is obtained in which the desired keto compound is not or hardly present.

In the oxidation process according to the invention various compounds may be started from, for instance compounds in which the group R in the general formula represents a phenyl, naphthyl, pyridyl, furyl, or thienyl group, which groups can optionally be substituted with, for instance, one or more substituents from the group consisting of Cl, $NO_2$, alkoxy with 1—8 C atoms and alkyl with 1—8 C atoms. As ester of the hydroxy acid, various esters can be applied, for instance the esters with, in the ester group, an alkyl group with 1—8 C atoms, a cycloalkyl group with 5—8 C atoms in the ring, a phenyl group or a naphthyl group. Alternatively an amide may be used.

In applying the process according to the invention the acid applied as solvent may be of a commercial grade and contain, for instance, 3% by weight of water. The quantity of acid may vary. Very suitable is a quantity of acid of 0.5—15 g per gramme of hydroxy derivative to be oxidized.

The catalytic quantity of cobalt required in the reaction mixture can be obtained by addition of a cobalt compound soluble in the reaction mixture, such as inorganic or organic cobalt salts, or by the formation of such a compound in situ. Preferably a cobalt salt of a saturated aliphatic monocarboxylic acid with 1—8 C atoms, specifically cobalt acetate (di- and/or trivalent cobalt), is dissolved in the reaction mixture, for instance in a quantity corresponding with 1—50 g cobalt per mole of compound to be oxidized. In addition to the cobalt salt, the bromide promotor, preferably an alkali metal bromide, is applied in a quantity of, for instance, 0.5—20 g bromide per mole of compound to be oxidized. The catalyst and promotor can be separated from the reaction mixture obtained and be used again if so desired.

In the oxidation according to the invention the pressure is as such not critical. Application of a pressure higher than atmospheric pressure may be an advantage in large scale oxidation, for instance if acetic acid is applied as solvent and air as oxygen-containing gas. The fact is that in such a case the explosive range of the

acetic acid vapour-nitrogen-oxygen system can be avoided by diluting the air with nitrogen or by applying a pressure higher than atmospheric.

The oxidation according to the invention can be carried out while applying methods known in the art for carrying out liquid phase oxidation with a molecular oxygen-containing gas, for instance by passing the gas through the reaction mixture for some time at the desired temperature, while the mixture is well stirred. In this process different times may be chosen during which the reaction conditions are maintained.

The oxidation according to the invention will be further elucidated in the following examples.

Example I

Into a cylindrical reaction vessel with a capacity of 250 ml, provided with 4 baffle plates, stirrer, reflux condenser and air inlet tube, 4 g cobalt (II) acetate.$4H_2O$, 0.41 g sodium bromide, 17 g ethyl mandelate (0.094 mole) and 116 ml acetic acid (99.5% by weight) were brought.

The reaction mixture was heated to 90°C, after which air was passed in at a rate of 20.5 l/hour. After the air had been passed in for 5 minutes, the temperature of the reaction mixture was 103°C. After it had been passed in for 25 minutes, the temperature of the reaction mixture was 97°C, and the passing in of air was discontinued.

The reaction mixture obtained was subjected to a gas chromatographic analysis, which showed that the ethyl mandelate was completely converted, with a selectivity of 96%, into the corresponding keto compound.

The reaction mixture was subsequently poured out into 1 l of water, and the aqueous solution obtained extracted with ether. The ether extract was neutralized with $NaHCO_3$ to a pH of about 8 and dried over $MgSO_4$. After evaporation of the ether under reduced pressure, 15.7 g light yellow product remained, which was the desired keto compound with a purity of 99% according to a gas chromatographic analysis. The yield was 92% of the yield theoretically possible.

Comparative examples

In the same way as described in example I it was tried to oxidize mandelic acid. After air had been passed through for 2 hours and 15 minutes, the reaction mixture was analyzed, which showed that the mandelic acid had not been converted.

Oxidation of the nitrile of mandelic acid (benzaldehyde-cyanohydrin) in the manner as described in example I did not result in a keto compound. After air had been passed through for 15 minutes, the reaction mixture was found to contain, beside the initial product, benzaldehyde only, while, after air had been passed through for 1 hour, benzoic acid had been formed as well.

Example II

In the manner as described in example I, 16.6 g methyl mandelate (0.1 mole) was oxidized. After air had been passed in for 1 hour, a 100% conversion was found to have been reached, and the selectivity was 94%.

Example III

Example I was repeated. The reaction temperature, however, was kept at 90°C. After air had been passed through for 33 minutes, a 100% conversion was found to have been reached, and the selectivity was 98%.

Example IV

Repetition of example I at a temperature of 110° had as a result that, after air had been passed through for 22 minutes, a 100% conversion had already been reached, and the selectivity was 98%.

Example V

In the same way as described in example I, 0.1 mole of mandelic acid amide was oxidized. After air had been passed through for 30 minutes, the conversion was 100% and the selectivity with respect to the corresponding keto-amide 81%.

Example VI

In the manner as described in example I, 0.25 mole ethyl mandelate was oxidized in 116 ml acetic acid with 4 g cobalt(II)acetate.$4H_2O$ and 0.41 g NaBr at a temperature of 100°C. After a period of 75 minutes a conversion of 100% was reached, and the selectivity was 98%.

Example VII

In the manner described in example I, 18 g ethyl mandelate (0.1 mole) was oxidized. After air had been passed through for 30 minutes, the conversion was 100%, and the reaction mixture obtained was further processed as follows:

First the acetic acid was distilled off under reduced pressure. Subsequently 100 g water was added to the remaining residue, and the mixture was subjected to extraction with ether. The organic layer obtained in this process was washed with water and neutralized with $NaHCO_3$ to a pH of 8. After drying of the organic layer over $MgSO_4$, the ether was evaporated under reduced pressure. 17.3 g of the keto compound was obtained (yield 95%), which, according to a gas chromatographic analysis, had a purity of 98.6%.

Example VIII

The aqueous layer obtained in example VIII during the extraction with ether, which layer contained the cobalt catalyst and the NaBr, was evaporated to dryness. To the residue of 4.7 g 18 g ethyl mandelate (0.1 mole) and 116 ml acetic acid (99.5% by weight) were added, upon which the ethyl mandelate was oxidized in the manner described in example I. After air had

been passed through for 45 minutes, the reaction mixture was further processed in the manner described in example VIII. 17.3 g keto compound was obtained (yield 92%), which, according to a gas chromatographic analysis, had a purity of 95%.

Example IX
In the manner described in example I, 0.1 mole 2,4-dichloroethylmandelate was oxidized. After air had been passed through for 20 minutes, the conversion was 100% and the selectivity with respect to the keto compound 93%. The keto compound obtained had a boiling point of 118°C at a pressure of 27 Pa.

Example X
In the manner described in example I, 0.1 mole 4-methoxy-ethylmandelate was oxidized. After air had been passed through for, 20 minutes, the conversion was 100% and the selectivity with respect to the keto compound 97%.

Example XI
In the manner described in example I, 0.1 mole ethyl ester of (2-thienyl)hydroxyacetic acid was oxidized. After air had been passed through for 30 minutes, a conversion of 100% was reached. After further processing of the reaction mixture 16.4 g product was obtained containing 96% of the relative keto compound (yield 87%).

Example XII
In the manner described in example I, 0.1 mole ethyl ester of (3-pyridyl)hydroxyacetic acid was oxidized. After air had been passed through for 2 hours and 20 minutes, the conversion was 97% and the selectivity with respect to the relative keto compound 82%. The keto compound obtained had a boiling point of 90°C at a pressure of 7 Pa.

Example XIII
In the manner described in example I, 0.1 mole ethyl ester of 4-nitromandelic acid was oxidized. After air had been passed through for 2 hours, a conversion of 96% was reached. The selectivity with respect to the relative keto compound was 60%. As byproduct the reaction mixture was found to contain 4-nitrobenzoic acid.

Example XIV
In the manner described in example I, 0.1 mole n-butyl mandelate was oxidized. After air had been passed through for 15 minutes, 100% conversion was reached. The selectivity with respect to the relative keto compound was 97%.

Example XV
In the manner described in example I, 0.1 mole 4-carbomethoxymethyl mandelate was oxidized. When air had been passed through for 40 minutes, 100% conversion was reached. The selectivity with respect to the corresponding keto compound was 93%.

Example XVI
In the manner described in example I, 0.1 mole 4-ethoxy-ethyl mandelate was oxidized. When air had been passed through for 30 minutes, 100% conversion was reached. The selectivity with respect to the desired keto compound was 92%.

Example XVII
In the manner described in example I, 0.1 mole 3-phenoxy-ethyl mandelate was oxidized. When air had been passed through for 30 minutes, 100% conversion was reached. The selectivity with respect to the keto compound concerned was 98%.

Example XVIII
In the manner described in example I, 0.1 mole ethyl mandalate in 110 ml acetic acid was oxidized at a temperature of 100°C with the aid of 2.7 g $CoBr_2 . 6H_2O$.

After air had been passed through for 35 minutes, 100% conversion was reached. The selectivity with respect to the keto compound concerned was 94%.

## Claims

1. Process for the preparation of substituted glyoxylic acid derivatives by oxidation of the corresponding hydroxy derivative, characterized in that an amide or ester of a hydroxy acid with the general formula of

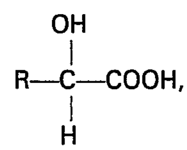

where R represents a possibly substituted aryl or heteroaryl group, is oxidized at a temperature of 70—150°C in a solvent, containing cobalt as catalyst and a bromide as promotor and using a saturated aliphatic monocarboxylic acid with 2—8 C atoms as the solvent, with a molecular oxygen-containing gas, and the corresponding amide or the corresponding ester of a glyoxylic acid with the general formule of

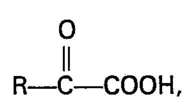

where R has the above-mentioned meaning, is recovered from the reaction mixture obtained.

2. Process according to claim 1, characterized in that, as bromide, an alkali metal bromide is applied.

**Patentansprüche**

Verfahren zur Herstellung von substituierten Glyoxylsäurederivaten durch Oxidation des entsprechenden Hydroxyderivats dadurch gekennzeichnet, daß man ein Amid oder einen Ester einer Hydroxysäure mit der allgemeinen Formel

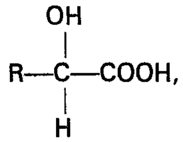

worin R eine gegebenenfalls substituierte Aryl- oder Heteroarylgruppe darstellt, bei einer Temperatur von 70—150°C in einem Lösungsmittel, das Kobalt als Katalysator und ein Bromid als Promotor enthält, und unter Verwendung einer gesättigten aliphatischen Monocarbonsäure mit 2—8 C-Atomen als Lösungsmittel mit einem molekularen Sauerstoff enthaltenden Gas oxidiert und das entsprechende Amid oder den entsprechenden Ester einer Glyoxylsäure mit der allgemeinen Formel

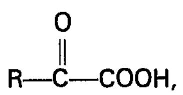

worin R die obige Bedeutung hat, aus dem erhaltenen Reaktions-gemisch gewinnt.

2. Verfahren nach Anspruch 1, dadurch gekennzeichnet, daß als Bromid ein Alkalimetallbromid eingesetzt wird.

**Revendications**

1. Procédé pour la préparation de dérivés substitués de l'acide glyoxylique par oxydation des dérivés hydroxy correspondants, caractérisé en ce qu'un amide ou un ester d'un acide hydroxy de formule générale

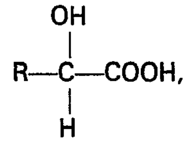

dans laquelle R représente un groupe aryle ou hétéroaryle qui peut être substitué, est oxydé à une température de 70—150°C dans un solvant, contenant du cobalt comme catalyseur et un bromure comme agent promoteur et en utilisant un acide monocarboxylique aliphatique saturé, ayant 2—8 atomes de carbone comme solvant, avec un gaz contenant de l'oxygène moléculaire, et l'amide correspondant ou l'ester correspondant d'un acide glyoxylique de formule générale

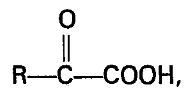

dans laquelle R a la signification mentionnée ci-dessus, est récupéré à partir du mélange réactionnel obtenu.

2. Procédé selon la revendication 1, caractérisé en ce qu'on utilise, comme bromure, un bromure de métal alcalin.